# EUROPÄISCHE PATENTSCHRIFT

(11) **EP 3 154 343 B1**
(45) Veröffentlichungstag und Bekanntmachung des Hinweises auf die Patenterteilung: **25.10.2023**
(21) Anmeldenummer: 15729428.1
(22) Anmeldetag: 11.06.2015
(51) Int. Cl.: A01N 25/00, A01P 11/00, A01N 43/44, A01N 43/52, A01N 47/44, A01N 43/84, A01N 47/18, A01N 43/90, A01N 43/40, A01N 43/62, A01N 63/10, A01N 37/46

(54) **SCHADNAGETIERKÖDER ENTHALTEND ZWEI ODER MEHR BLUTGERINNUNGSHEMMENDE VERBINDUNGEN**
RODENT BAIT CONTAINING TWO OR MORE ANTICOAGULATING COMPOUNDS
APPÂT POUR RONGEURS CONTENANT DEUX OU PLUS COMPOSÉS ANTICOAGULANTS

(30) Priorität: 11.06.2014 DE 102014108210
(43) Veröffentlichungstag der Anmeldung: 19.04.2017
(73) Patentinhaber: Gulba, Dietrich, 45133 Essen (DE)
(72) Erfinder: Gulba, Dietrich, 45133 Essen (DE)
(74) Vertreter: Ullrich & Naumann PartG mbB
(86) Internationale Anmeldenummer: PCT/EP2015/063056
(87) Internationale Veröffentlichungsnummer: WO 2015/189331

(56) Entgegenhaltungen:
- WO-A1-01/87879
- WO-A1-2010/043322
- WO-A2-2008/037966
- AU-B2- 508 245
- US-A- 4 011 332
- . .: "Abstracts from the 21st International Congress on Thrombosis", Pathophysiol Haemos Thromb, 1 January 2010 (2010-01-01), pages A73-A116, XP55627042, Basel, Switzerland DOI: https://doi.org/10.1159/000318097 Retrieved from the Internet: URL:https://www.karger.com/Article/Pdf/318 097 [retrieved on 2019-09-27]
- Jorge Labrador ET AL: "Management of Bleeding Complications of Dabigatran", Journal of Hematology & Thromboembolic Diseases, vol. 02, no. 01, 1 January 2013 (2013-01-01), pages 1-4, XP55627048, DOI: 10.4172/2329-8790.1000127

## Beschreibung

### TECHNISCHES GEBIET

Die vorliegende Offenbarung betrifft einen Schadnagetierköder. Der Schadnagetierköder enthält zwei oder mehr Verbindungen ausgewählt aus der Gruppe bestehend aus den Klassen Thrombininhibitoren, Thrombinrezeptor-Antagonisten, Faktor Xa-Inhibitoren, Antagonisten des ADP-Rezeptors P2Y12 und Antagonisten des GPIIb/IIIa-Rezeptors.

### HINTERGRUND

Die folgende Diskussion des Hintergrunds der Offenbarung wird lediglich bereitgestellt, um dem Leser das Verstehen zu erleichtern, und ist kein Eingeständnis, Stand der Technik der vorliegenden Erfindung zu beschreiben oder darzustellen.

Wildnagetiere stellen seit jeher ein erhebliches Problem für die Gesundheit des Menschen, für Eigentum und für die Nahrungsmittelversorgung dar. Schon zur Zeit der Pharaonen wurden Katzen gegen Nager eingesetzt. Allein von der Ratte sind fast 70 Krankheiten bekannt, von denen viele auf den Menschen übertragen werden können wie die Beulenpest, Typhus oder Morbus Weil. In der Landwirtschaft sind Wildnagetiere eine ökonomische Bedrohung. Nicht nur der Verzehr von Futter- oder Nahrungsmitteln, sondern vor allem auch Kontamination durch Kotballen und Urin führen zu wirtschaftlichem Schaden. Es wird geschätzt, dass etwa 10 % der weltweiten Nahrungsmittelversorgung allein durch Ratten verbraucht wird oder Schaden erleidet. Auch Krankheiten von Nutztieren wie Maul- und Klauenseuche oder Schweinepest werden von Wildnagetieren übertragen. Daneben kommt es auch zu weiteren Schäden an Gebäuden und Einrichtungen, da Wildnager beispielsweise auch Wasser- und Abwasserleitungen oder Kabel beschädigen können.

Das bei weitem verbreitetste Verfahren zur Bekämpfung von Wildnagetieren ist der Einsatz von Fraßködern - im Einzelfall werden auch Lagerräume und/oder unterirdische Nagetiergänge begast. An geeignete Wirkstoffe für Fraßköder bestehen besondere Anforderungen. So leben Ratten in Gruppen, zeigen ausgeprägtes Sozialverhalten und ein gutes Gedächtnis. Junge Männchen stellen sich freiwillig als Vorkoster, während die übrigen Ratten für die nächsten Stunden abwarten. Stirbt der Vorkoster innerhalb von zwei Tagen, weil er von einem vergifteten Köder gefressen hat, rühren seine Artgenossen diesen nicht mehr an. Ein als Rodentizid geeigneter Wirkstoff muss somit einen entsprechend verzögerten Wirkungseintritt zeigen, sodass Artgenossen des Vorkosters nicht vom Verzehr eines entsprechenden Köders abgehalten werden. Die heute für Fraßköder verwendeten Rodentizide sind üblicherweise Antikoagulantien, da Zinkphosphid, früher typischerweise in Giftweizen verwendet, Arsenverbindungen, Bariumcarbonat, Strychnin und weißer Phosphor seit geraumer Zeit nicht mehr als Rodentizide zugelassen sind.

US 4,011,332 A offenbart rodentizide Zusammensetzungen enthaltend als wirksame Komponenten a) Hemmer der Blutgerinnung, Acenocoumarol, oder Coumatetralyl und b) Induktoren vom hämorrhagischen Läsionen in einer Köderformulierung mit einem Trägermaterial.

AU 508 245 B2 offenbart die Verwendung von Ködern auf der Basis von Diphacinon als Rodentizid. Die Köderformulierung enthält zusätzlich zum Trägermaterial noch Cholecalciferol.

Van Ryn et al. 2010 ("Abstracts from the 21st International Congress on Thrombosis", Pathophysiol Haemos Thromb, 1. Januar 2010, Seite A94: Abstract P486) offenbaren, dass es bei einer Überdosierung von Thrombininhibitoren, wie beispielsweise Dabigatran, zu Blutungen kommen kann. Dies wurde in Kaninchen nachgewiesen.

Labrador et al. 2013 ("Management of Bleeding Complications of Dabigatran", Journal of Hematology & Thromboembolic Diseases, Bd. 02, Nr. 01, 1. Januar 2013, Seiten 1-4) offenbaren Untersuchungen zur Behandlung von nach Überdosierung von Dabigatran auftretenden Blutungen in Ratten. Derartige Blutungen können beispielsweise durch die Gabe hoher Dosen eines Konzentrats von aktiviertem Prothrombin-Komplex oder von rekombinantem Faktor VIIa gestoppt werden.

Ein erhebliches Problem der Antikoagulantien ist jedoch mittlerweile, neben Persistenz und/oder Bioakkumulation, eine verbreitete Resistenz bei Wildnagetieren. Eine Resistenz gegen ein Antikoagulans wurde bereits in den 50er Jahren gefunden. Nachdem zunächst nur die Antikoagulantien der ersten Generation wie Warfarin und Cumatetralyl betroffen waren, wurden später auch Resistenzen gegen die wirksameren Antikoagulantien der zweiten Generation wie Difenacoum oder Bromadiolon beobachtet. Mittlerweile sind mehr als 40% aller Rattenstämme gegen die Antikoagulantien resistent.

Versuche, andere Wirkstoffe als Alternativen einzusetzen, waren bislang ohne Erfolg. So könnten Derivate des Benzocains (4-Aminobenzoesäureethylester), eines Lokalanästhetikums, durch Bildung von Methämoglobin zur Bekämpfung von Wildnagern in Frage kommen. Methämoglobin kann keinen Sauerstoff binden und verändert Hämoglobin in seiner Umgebung dahingehend, dass dieses nur noch Sauerstoff aufnehmen, aber nicht mehr abgeben kann. *In vivo* Versuche an Ratten zeigten jedoch nicht den erhofften Erfolg (Conole, D., et al., Bioorg. Med. Chem. (2014) 22: 2220-2235).

Seit dem 1. Januar 2013 wäre ein Vertrieb herkömmlicher Rodentizide in der Europäischen Union eigentlich auf Grund des Überschreitens der als für Organismen unbedenklich vorhergesagten Konzentration in der Umwelt nicht mehr zulässig. Mangels Alternativen besteht jedoch weiterhin eine befristete Zulassung zur Anwendung durch sachkundige Personen.

Es besteht somit Bedarf an einem effektiven Weg zur Bekämpfung von Wildnagetieren.

### ZUSAMMENFASSUNG

Die vorliegende Offenbarung kann allgemein dahingehend aufgefasst werden, dass sie der Bekämpfung von Wildnagetieren dient. Dabei werden Schadnagetierköder bereitgestellt, die auf dem Einsatz hier offenbarter Wirkstoffe beruhen. Dabei ist ein eingesetzter Wirkstoff so ausgewählt, dass er mit einer Verwendung in einem Köder kompatibel ist, indem der Wirkungseintritt des Wirkstoffs auf das Verhalten von Wildnagetieren abgestimmt ist. Darüber hinaus ist ein eingesetzter Wirkstoff im Allgemeinen so ausgewählt, dass er bestehende Resistenzen und die Entwicklung neuer Resistenzen vermeidet.

Erfindungsgemäss wird ein Schadnagetierköder, enthaltend zwei oder mehr Verbindungen ausgewählt aus der Gruppe bestehend aus den Klassen Thrombininhibitoren, Thrombinrezeptor-Antagonisten, Faktor Xa-Inhibitoren, Antagonisten des ADP-Rezeptors P2Y12 und Antagonisten des GPIIb/IIIa-Rezeptors,
wobei der Thrombininhibitor wenigstens eine Verbindung ausgewählt aus der Gruppe bestehend aus Melagatran, Ximelagatran, Dabigatran, Dabigatranetexilat und Argatroban ist,
wobei der Thrombinrezeptor-Antagonist wenigstens eine Verbindung ausgewählt aus der Gruppe bestehend aus Atopaxar und Vorapaxar ist,
wobei der Inhibitor von Faktor Xa wenigstens eine Verbindung ausgewählt aus der Gruppe bestehend aus Rivaroxaban, Apixaban, Edoxaban, Betrixaban, Darexaban und Otamixaban ist,
wobei der Antagonist des ADP-Rezeptors P2Y12 wenigstens eine Verbindung ausgewählt aus der Gruppe bestehend aus Ticlopidin, Clopidogrel, Prasugrel und Ticagrelor ist,
wobei der Antagonist des GPIIb/IIIa-Rezeptors wenigstens eine Verbindung ausgewählt aus der Gruppe bestehend aus Eristicophin, Cotiarin, Crotatroxin, Cerastin, Durissin, Horridin, Ruberin, Lachesin, Basilicin, Lutosin, Molossin, Oreganin, Viridin, Tergeminin, Barbourin, Eptifibatid, Orbofiban, Roxifiban, Sibrafiban und Tirofiban ist, bereitgestellt.

Die im Vorangehenden beschriebene Zusammenfassung ist nicht einschränkend und weitere Merkmale und Vorteile der hier beschriebenen Schadnagetierköder werden aus der folgenden ausführlichen Beschreibung und den Patentansprüchen ersichtlich werden.

### AUSFÜHRLICHE BESCHREIBUNG

Soweit nicht anders angegeben oder aus dem Zusammenhang eindeutig eine andere Bedeutung ersichtlich ist, haben die folgenden Begriffe und Ausdrücke, wenn sie in diesem Dokument, inklusive Beschreibung und Patentansprüchen, verwendet werden, die im Folgenden angegebenen Bedeutungen.

Die Begriffe "Behandeln" und "Behandlung" wie in diesem Dokument verwendet, betreffen sowohl eine prophylaktische oder präventive Maßnahme als auch eine Maßnahme, die eine therapeutische Wirkung entfaltet. "Behandeln" und "Behandlung" bezeichnen beispielsweise das Verhindern, das Verlangsamen, das Vermindern, oder das zumindest teilweise Mildern oder Aufheben eines unnormalen, inklusive eines pathologischen, Zustands in einem Organismus eines Subjekts. Der Ausdruck "unnormaler Zustand" bezieht sich in der Regel auf die Funktion einer Zelle, eines Organs oder eines Gewebes in einem Organismus, die von der üblichen Funktion in einem entsprechenden Organismus abweicht. Ein unnormaler Zustand kann beispielsweise mit einer veränderten Zellproliferation, Zelldifferenzierung oder Überlebensdauer einer Zelle zusammenhängen. Zu Behandlungsbedürftigen zählen Subjekte, die bereits von einer Erkrankung und/oder Störung betroffen sind, als auch jene, die anfällig für das Auftreten der Erkrankung / Störung sind oder jene, in denen das Auftreten der Erkrankung / Störung verhindert werden soll. Üblicherweise reduziert, stabilisiert oder verhindert eine Behandlung das Fortschreiten eines Symptoms, das mit dem Vorliegen und/oder Voranschreiten einer Erkrankung oder eines pathologischen Zustands einhergeht. Der Begriff "Behandeln" betrifft typischerweise ein Verfahren, bei dem eine Verbindung Zellen oder Gewebe eines Subjekts verabreicht wird.

Die Begriffe "Bekämpfen" und "Bekämpfung" wie in diesem Dokument verwendet betreffen eine Maßnahme, mit der erreicht wird, dass ein unerwünschter Organismus, im vorliegenden Fall ein Schadnagetier, abgetötet wird. "Bekämpfung" hat in einigen Ausführungsformen eine dem Begriff "Kontrolle" entsprechende Bedeutung. "Bekämpfen" und "Bekämpfung" bezeichnen beispielsweise das Hervorrufen, das Beschleunigen, das Fördern, inklusive das Ermöglichen des Auftretens eines unnormalen, inklusive eines pathologischen, Zustands in einem Organismus eines Schadnagetiers. Typischerweise zählt zum "Bekämpfen" ein Verfahren und/oder eine Verwendung, bei dem/der eine Verbindung Zellen oder Gewebe eines Schadnagetiers zugeführt wird. Die Begriffe "Bekämpfen" und "Bekämpfung" beinhalten daneben in der Regel auch solche Merkmale der Verwendung des Schadnagetierköders, die es erlauben, dass eine entsprechende Verbindung Zellen oder Gewebe eines Schadnagetiers zugeführt werden kann. In den hier offenbarten Verwendungen beruhen solche Merkmale, die ein Verabreichen ermöglichen, typischerweise auf dem zeitlichen Rahmen des Wirkungseintritts der in einer solchen Verwendung des Schadnagetierköders eingesetzten Wirkstoffe.

Der Ausdruck "bestehend aus" wie in diesem Dokument verwendet, bedeutet einschließend und begrenzt auf das, was dem Begriff "bestehend aus" folgt. Der Begriff "bestehend aus", gibt somit an, dass aufgeführte Elemente erforderlich oder notwendig sind und dass keine weiteren Elemente vorhanden sein dürfen. Der Begriff "im Wesentlichen bestehend aus" wird dahingehend verstanden, dass er bedeutet, dass jedwede Elemente, die nach dem Ausdruck definiert sind, umfasst sind und dass weitere Elemente, beispielsweise in einer Probe oder einer Zusammensetzung zugegen sein können, die die Aktivität oder Wirkung, die für die betreffenden Elemente in diesem Dokument angegeben sind, nicht verändern, also sie nicht beeinträchtigen und nicht zu ihr beitragen. Als Beispiel bedeutet der Begriff für eine als Rodentizid wirksame Zusammensetzung, dass diese Trägerstoffe/ Hilfsmittel enthalten kann, wenn sie im Wesentlichen aus einem oder mehreren Wirkstoffen besteht. Somit gibt der Ausdruck "im Wesentlichen bestehend aus" an, dass die definierten Elemente notwendig oder erforderlich sind, dass aber weitere Elemente optional sind und zugegen sein können oder nicht, je nachdem, ob sie für die Wirkung oder Wirksamkeit der definierten Elemente von Belang sind oder nicht.

Das Wort "etwa" bezieht sich wenn hier verwendet auf einen Wert, der für einen bestimmten Wert, wie von einem Durchschnittsfachmann bestimmt, innerhalb eines akzeptablen Fehlerbereichs liegt. Dies wird teilweise davon abhängig sein, wie der jeweilige Wert ermittelt oder gemessen worden ist, d.h. von den Einschränkungen des Messsystems. "Etwa" kann beispielsweise innerhalb einer Standardabweichung von 1 oder mehr bedeuten, je nach Gebrauch im jeweiligen Gebiet. Der Begriff "etwa" wird auch verwendet um anzugeben, dass der Betrag oder Wert der bezeichnete Wert sein kann oder ein anderer Wert, der näherungsweise gleich ist. Der Begriff soll ausdrücken, dass ähnliche Werte gleichwertige Ergebnisse oder Wirkungen, wie in diesem Dokument offenbart, begünstigen. In diesem Zusammenhang kann "etwa" sich auf einen Bereich von bis zu 10 % über und/oder unter einem bestimmten Wert beziehen. In einigen Ausführungsformen bezieht "etwa" sich auf einen Bereich von bis zu 5 % über und/oder unter einem bestimmten Wert, wie etwa 2 % über und/oder unter einem bestimmten Wert. In einigen Ausführungsformen bezieht "etwa" sich auf einen Bereich von bis zu 1 % über und/oder unter einem bestimmten Wert. In einigen Ausführungsformen bezieht "etwa" sich auf einen Bereich von bis zu 0,5 % über und/oder unter einem bestimmten Wert. In einer Ausführungsform bezieht sich "etwa" auf einen Bereich von bis zu 0,1 % über und/oder unter einem bestimmten Wert.

Der Konjunktionalausdruck "und/oder" zwischen mehreren Elementen, wenn hier verwendet, wird als sowohl individuelle als auch kombinierte Optionen umfassend verstanden. Sind beispielsweise zwei Elemente durch "und/oder" verknüpft, betrifft eine erste Option den Einsatz des ersten Elements ohne das zweite. Eine zweite Option betrifft den Einsatz des zweiten Elements ohne das erste. Eine dritte Option betrifft den Einsatz des ersten und des zweiten Elements zusammen. Es wird verstanden, dass jede beliebige dieser Optionen unter die Bedeutung des Ausdrucks fällt und somit die Bedingungen des Begriffs "und/oder", wie in diesem Dokument verwendet, erfüllt.

Der Begriff "niedermolekular" im Zusammenhang mit einer Verbindung, beispielsweise als ein niedermolekularer Thrombininhibitor, bezieht sich auf eine molekulare Masse, die im Bereich bis etwa 5000 Da liegt. In einigen Ausführungsformen kann die Masse einer niedermolekularen Verbindung im Bereich bis etwa 2000 Da liegen.

Der Begriff "Prodrug" bezeichnet eine Verbindung, die im Organismus eines Tieres wie z.B. eines Nagetieres - beispielsweise auf enzymatischem, mechanischem und/oder elektromagnetischem Weg - in seine aktive Form überführt wird, die die gewünschte pharmakologische oder toxikologische Wirkung zeigt. Ein "Prodrug" ist demgemäß ein Derivat des aktiven Wirkstoffs, das selbst noch pharmakologisch/toxikologisch inaktiv ist oder im Vergleich zum endgültigen aktiven Wirkstoff eine geringere Wirkung zeigt. Prodrugs werden typischerweise eingesetzt, um Herausforderungen hinsichtlich Stabilität, Spezifität, Toxizität oder Bioverfügbarkeit zu bewältigen. Ein Prodrug kann beispielsweise im Vergleich zum endgültigen aktiven Wirkstoff eine vorteilhafte Löslichkeit, Gewebekompatibilität oder Freisetzung aufweisen. Beispielsweise kann ein Prodrug im Vergleich zum endgültigen aktiven Wirkstoff eine Schutzgruppe an einer funktionellen Gruppe tragen, die *in vivo* durch Solvolyse oder enzymatisch entfernt wird. Als weiteres Beispiel kann ein Prodrug *in vivo* über eine Oxidation und/oder über eine Phosphorylierung oder Glykosylierung in einen endgültigen aktiven Wirkstoff überführt werden. Dabei können ein oder mehrere Enzyme und/oder Magensäure beteiligt sein. Zu Beispielen typischer Prodrugs zählen Carbonsäurederivate wie ein Ester, der durch Reaktion einer Stamm-Säureverbindung mit einem geeigneten Alkohol, z.B. ein C₁₋₆-Alkohol erhalten wird, ein Amid, das durch Reaktion einer Stamm-Säureverbindung mit einem geeigneten Amin, z.B. ein C₁₋₆-Amin erhalten wird oder eine acylierte basische Gruppe wie z.B. ein C₁₋₆-Acylamin, die durch Reaktion einer base-haltigen Stammverbindung mit einer Carbonsäureverbindung erhalten wird.

Der Begriff "Verabreichen" bezieht sich wenn hier verwendet auf jedwede Art des Transferierens, Zuführens, Einführens oder Transportierens von Material wie einer Verbindung, z.B. einer pharmazeutischen Verbindung, oder eines anderen Reagenzes wie eines Antigens in/zu einem Subjekt. Zu Verabreichungsformen zählen beispielsweise orale Verabreichung, topischer (örtlicher) Kontakt, intravenöse, intraperitoneale, intramuskuläre, intranasale und subkutane Verabreichung. In den hier beschriebenen Anwendungen und Verfahren erfolgt die Verabreichung an Nagetiere typischerweise oral. Verabreichung "in Kombination mit" einem oder mehreren weiteren Stoffen wie z.B. einem oder mehreren pharmazeutischen Wirkstoffen umfasst simultane, also gleichzeitige, und aufeinander folgende Verabreichung in jeder Reihenfolge. Beim Ausbringen eines Köders als Verabreichen wird insofern dem Sozialverhalten von Nagetieren Rechnung getragen, als den Nagetieren für die Aufnahme Zeitpunkt und Reihenfolge innerhalb der Gruppe überlassen bleiben.

Ein für ein Nagetier verträgliches Salz oder eine für ein Nagetier verträgliche Darreichungsform ist ein Salz bzw. eine Darreichungsform, von der keine unmittelbaren negativen Wirkungen an einem Nagetier ausgehen. Ein solches Salz bzw. eine solche Darreichungsform lässt somit insbesondere bei der Ratte keinen Rückschluss des Nagetiers auf mögliche nachteilige Wirkungen eines Köders oder eines andersartigen Angebots eines Wirkstoffs zu. In einigen Ausführungsformen sind ein für ein Nagetier verträgliches Salz oder eine für ein Nagetier verträgliche Darreichungsform ein pharmazeutisch verträgliches Salz oder eine pharmazeutisch verträgliche Darreichungsform.

Eine "wirksame Menge" eines Gegenstandes, wie einer Verbindung, ist eine Menge - entweder als Einzeldosis oder als Teil einer Reihe von Dosen - die beim eingesetzten Dosierungsschema die gewünschte therapeutische oder toxische Wirkung erzielt, das heißt, ein bestimmtes physiologisches Ziel wie das Hemmen eines Enzyms bewirkt. Vorliegend kann ein beabsichtigtes Ziel im Ableben eines Nagetiers bestehen. Die Dosierung hängt von verschiedenen Faktoren ab, einschließlich Art und Größe des Nagetiers und der Art der Anbietung oder Verabreichung und anderen Faktoren.

Singularformen wie "eine", "ein", "der", "die" oder "das" schließen die Pluralform ein, wenn sie in diesem Dokument verwendet werden. So bezeichnet beispielsweise eine Bezugnahme auf "eine Zelle" sowohl eine individuelle Zelle als auch eine Mehrzahl an Zellen. In einigen Fällen wird explizit der Ausdruck "ein oder mehrere" verwendet, um im jeweiligen Fall darauf hinzuweisen, dass die Singularform die Pluralform mit umfasst. Derartige explizite Hinweise schränken die allgemeine Bedeutung der Singularform nicht ein. Falls nicht anders angegeben, werden die Begriffe "zumindest", "mindestens" und "wenigstens", wenn sie eine Abfolge von Elementen vorangehen, dahingehend verstanden, dass sie sich auf jedes dieser Elemente beziehen. Die Begriffe "zumindest ein", "mindestens ein(e)", "wenigstens einer" oder "wenigstens eine(r) von" schließen beispielsweise ein, zwei, drei, vier oder mehr Elemente ein.

Der Ausdruck "zumindest im Wesentlichen bestehend aus" wird, wenn hier verwendet, als die Begriffe "im Wesentlichen bestehend aus" und "bestehend aus" umfassend verstanden. Der Begriff "zumindest im Wesentlichen bestehend aus", gibt somit an, dass in einigen Ausführungsformen aufgeführte Elemente erforderlich oder notwendig sind und dass keine weiteren Elemente vorhanden sein dürfen. Der Begriff "zumindest im Wesentlichen bestehend aus", gibt somit auch an, dass in einigen Ausführungsformen aufgeführte Elemente erforderlich oder notwendig sind, dass aber weitere Elemente optional sind und zugegen sein können oder nicht, je nachdem, ob sie für die Wirkung oder Wirksamkeit der definierten Elemente von Belang sind oder nicht. Es wird weiterhin verstanden, dass geringe Abweichungen über oder unter einen hier angegebenen Bereich eingesetzt werden können, um ein im Wesentlichen gleiches Ergebnis zu erzielen wie ein Wert, der innerhalb des Bereichs liegt. Falls nicht anders angegeben, ist die Offenbarung eines Bereichs auch als kontinuierlicher Bereich vorgesehen, inklusive aller Einzelwerte, die zwischen dem Minimal- und dem Maximalwert liegen.

Ein hier offenbarter Schadnagetierköder greift in der Regel in die Vorgänge der Blutgerinnung ein. Dabei wird im Allgemeinen die Blutgerinnung gehemmt. Diese Wirkung ist dem Grunde nach bekannt und wird therapeutisch zur Vermeidung von Blutgerinnseln eingesetzt. Gefürchtete Folgen von Blutgerinnseln sind zum Beispiel Herzinfarkt, Schlaganfall oder Lungenembolie. Eine solche therapeutische Wirkung geht mit dem Risiko von Blutungen einher. Diese am Menschen unerwünschte Arzneimittelwirkung als Nebenwirkung ist beim Bekämpfen von Nagetieren, insbesondere der Ratte, in Form einer nicht unmittelbar eintretenden, aber dennoch tödlichen Blutung eine willkommene Wirkung.

Bei der Blutstillung adhärieren zunächst Plättchen (Thrombozyten) an Gewebestrukturen, aggregieren miteinander und bilden einen hämostatischen Pfropf. Ohne sich auf eine bestimmte Theorie zu beschränken, kann die Wirkung bei einer Verwendung des Schadnagetierköders gemäß der vorliegenden Offenbarung dahingehend verstanden werden, dass diese Aggregation und damit die Bildung eines hämostatischen Pfropfes verringert wird und ggf. unterbleibt. Gemäß diesem Verständnis ist eine Adhäsion der Plättchen in der Regel nicht der Angriffsort eines hier offenbarten Schadnagetierköders.

Bei der Blutstillung adhärieren aktivierte Plättchen, degranulieren und sezernieren im Rahmen der Aktivierung gerinnungsaktive Mikropartikel sowie ADP und Thromboxan A2. Diese binden an ihre Plättchenrezeptoren und führen zur Aktivierung weiterer Plättchen. Die Aggregation der Plättchen untereinander wird über die Bindung von Fibrinogen und Ca²⁺ an Rezeptoren auf den Plättchen vermittelt. In der sekundären Phase, der Fibrinbildungsphase, verfestigt gebildetes Fibrin den hämostatischen Pfropf. Die eigentliche Blutgerinnung ist somit die Umwandlung von löslichem Fibrinogen in unlösliches Fibrin. Die Aktivierung der Blutgerinnung mündet in einen Prothrombinasekomplex aus Faktor Xa, Faktor Va, Phospholipid und Ca²⁺. Das Schlüsselenzym bei der Blutgerinnung ist dabei die Protease Thrombin. Thrombin katalysiert die Umwandlung von Fibrinogen zu Fibrin, indem es vom Fibrinogen die Fibrinopeptide A und B abspaltet. Die dadurch entstandenen Fibrinmonomere aggregieren zu Polymeren.

Zur Vergiftung von Nagetieren, wie der Ratte, der Maus, der Wühlmaus, des Kaninchens, des Opossums und des Erdhörnchens werden bislang unter anderem Warfarin, Cumatetralyl (auch: Coumatetralyl), Diphacinon, Flocumafen (auch: Flocoumafen), Brodifacoum und Bromadiolon eingesetzt. Warfarin ist (RS)-4-Hydroxy-3-(3-oxo-1-phenyl-butyl)-cumarin und Cumatetralyl ist 4-Hydroxy-3-(1,2,3,4-tetrahydro-1-naphthyl)-cumarin. Difenacoum ist 3-(3-Biphenyl-4-yl-1,2,3,4-tetrahydro-1-naphthyl)-4-hydroxycumarin, Flocumafen ist 4-Hydroxy-3-[3-(4'-trifluormethylbenzyl-oxyphenyl)-1,2,3,4-tetrahydro-1-naphtyl]cumarin, Brodifacoum ist 3-(3-(4'-Brom-1,1'-biphenyl-4-yl)-1,2,3,4-tetrahydro-1-naphthyl)-4-hydroxycumarin, Bromadiolon 3-[3-(4'-Brombiphenyl-4-yl)-3-hydroxy-1-phenylpropyl]-4-hydroxycumarin. Es handelt sich also um Cumarine. Cumarine, Derivate des 4-Hydroxy-Cumarins oder 1,3-Indandions, sind Vitamin K-Antagonisten, indem sie die Enzyme Vitamin-K-Chinon-Reduktase und Vitamin-K-Epoxid-Reduktase blockieren.

Vitamin K ist als Kofaktor für die posttranslationale γ-Carboxylierung von N-terminalen Glutaminsäureresten in einer Reihe von Proteinen erforderlich, wozu auch die Gerinnungsfaktoren II, VII, IX und X sowie die Gerinnungsmodulatoren Protein C und Protein S zählen. Cumarine wie z.B. Phenprocoumon (Marcumar^{®}, Falithrom^{®}) oder Warfarin (Couxnadin^{®}) sind somit indirekt wirkende Antikoagulantien. Entsprechend den unterschiedlichen biologischen Halbwertszeiten Vitamin-K-abhängiger Gerinnungsfaktoren wird der maximale Cumarineffekt erst mit erheblicher Verzögerung manifest, im Menschen nach 24 bis 36 Stunden. Das Risiko beim Einsatz von Warfarin an menschlichen Patienten ist seine geringe therapeutische Breite. Bei Überdosierung treten Blutungen in Gastrointestinaltrakt, Gehirn, Nebenniere oder Netzhaut auf. Die optimale Dosis wird bevorzugt individuell festgelegt. Bei Anwendung als Rodentizid spielen derartige Erwägungen keine Rolle, vielmehr wird ein Erreichen einer letalen Dosis (LD) angestrebt.

Phenprocoumon wird am Menschen eingesetzt, um einer Thrombusbildung und daraus resultierenden Embolien vorzubeugen, beispielsweise zur Thrombose-Rezidivprophylaxe oder bei Vorhofflimmern. Eine gefürchtete unerwünschte Wirkung beim Einsatz am Menschen sind schwere Blutungen, die vor allem bei Überdosierung und gleichzeitigem Bluthochdruck entstehen können.

Zwei weitere bekannte Rodentizide sind Difenacoum (2-(Diphenylacetyl)-1H-inden-1,3(2H)-dion) Pindon (2-Pivaloyl-1,3-indandion). Als Indandion-Verbindungen wirken sie wie ein Cumarin als Vitamin K-Antagonisten.

Die Wirksamkeit von Cumarinen und Indandion-Verbindungen beruht auf der langsamen Akkumulation der Substanz im Tierkörper und der in der Folge zunehmenden Gerinnungshemmung weshalb die Tiere langsam innerlich verbluten. Gerade die nur progressiv einsetzende Giftwirkung machte die Cumarine und Indandion-Verbindungen als Schadnagergifte so besonders erfolgreich.

Bei der Aufnahme eines mit einem Cumarin oder einer Indandion-Verbindungen versetzten Köders bleiben die Ratten zunächst am Leben und zeigen keinerlei Vergiftungssymptome. Ein Wildnager wie eine Ratte, die sich regelmäßig von der Ködernahrung ernährt, stirbt somit nicht unmittelbar oder in kurzem Abstand nach Köderverzehr, sondern bleiben noch einige Tage am Leben bevor sie schließlich innerlich verblutetet. Dies gilt somit auch für eine "Vorkosterratte". Das protektive Sozialverhalten der Ratte wird damit wirksam umgangen und der Pestizid (Cumarin/Indandion)-haltige Nahrungsköder wird vom Rattenvolk nicht gemieden. Vielmehr ernährt sich das ganze Rattenvolk von dem leicht zu habenden Köder was in einer zwar zeitlich versetzten aber vollständigen Auslöschung der gesamten Rattenpopulation resultiert. Regelmäßig angebotene Köder, die eine Cumarin- oder Indandion-Verbindung aufweisen, haben so zuverlässig zur sicheren Elimination großer Rattenpopulationen geführt.

Mittlerweile ist durch den verbreiteten Einsatz von Cumarin- und Indandion-Verbindungen bei nahezu einem Drittel bis der Hälfte der weltweiten Rattenstämme mit einer ausgeprägten Resistenz gegen Cumarin- und Indandion-Verbindung zu rechnen. Diese Resistenz beruht auf einem ebenfalls vorhandenen alternativen Thiol- (SH-)/Disulfid(S-S) abhängigen Reduktionsweg des Vitamin K. Anders als beim Menschen, bei dem der thiolabhängige Reduktionsweg i.d.R. nur sehr ungenügend ausgebildet ist, existiert heute eine Vielzahl von Rattenstämmen, die diesen alternativen Reduktionsweg des Vitamin K aktivieren können. Beim dauerhaften Einsatz der Cumarine als Pestizide stellte der thiolabhängige Reduktionsweg für die Ratte einen signifikanten Selektionsvorteil dar. Über die vielen Jahrzehnte, in denen Cumarin-Köder sehr effizient verwendet wurden haben sich insbesondere die Cumarin-resistenten Stämme ungestört vermehren können, sodass mittlerweile eine deutliche Selektion dieser Cumarin-resistenten Rattenstämme entstanden ist.

Die hier offenbarten Schadnagetierköder beruhen auf der Beobachtung, dass neben Vitamin K-Antagonisten weitere Wirkstoffe mit antithrombotischer Wirkung einen ausreichend verzögerten Wirkungseintritt aufweisen, um als Rodentizid in Frage zu kommen.

Die hier offenbarten Schadnagetierköder sind selektiv für Nagetiere. Die zu Grunde liegenden Verbindungen zeigen in diesen Ausführungsformen nicht die gleiche fatale antithrombotische Wirkung an beispielsweise Vögeln.

In einigen Ausführungsformen betreffen die hier offenbarten Schadnagetierköder bevorzugt die Gattung der Ratte (*Rattus*), zu der beispielsweise die Hausratte (*Rattus rattus*), *Rattus andamanensis*, die Wanderratte (*Rattus norvegicus*), die Reisfeldratte (*Rattus argentiventer*) oder die Pazifische Ratte (*Rattus exulans*) zählen. Die zu Grunde liegenden Verbindungen zeigen in diesen Ausführungsformen nicht die gleiche fatale antithrombotischer Wirkung an beispielsweise Mäusen oder Hamstern. In einigen Ausführungsformen sind die hier offenbarten Schadnagetierköder selektiv für die Gattung der Ratte (*Rattus*)*.* Beispielsweise sind Inhibitoren von Faktor Xa wie Rivaroxaban (eine Verbindungen gemäß der Internationalen Patentanmeldung WO 01/47919) oder Apixaban (Eliquis^{®}; eine Verbindungen gemäß der Internationalen Patentanmeldung WO 03/026652) oder Betrixaban (N-(5-Chlorpyridin-2-yl)-2-([4-(N,N-dimethylcarbamimidoyl)benzoyl]amino)-5-methoxybenzamid; eine Verbindungen gemäß der Internationalen Patentanmeldungen WO 01/64642 und WO 01/64643) typischerweise selektiv für die Gattung *Rattus.*

Ein weiteres Beispiel für Verbindungen, deren Verwendung in der Regel zumindest großenteils selektiv für die Gattung *Rattus* ist, ist ein GPIIb/IIIa-Rezeptorantagonist. So betreffen Verwendungen von Inhibitoren gemäß der internationalen Patentanmeldungen WO 95/14683 und WO 93/19046 typischerweise selektiv die Gattung *Rattus.* Auch Verwendungen von Verbindungen gemäß dem US-amerikanischen Patent US 5,721,366 und dem Europäischen Patent EP 0 656 348 sind typischerweise selektiv für die Gattung der Ratte.

Typischerweise wird die als Rodentizid geeignete Verbindung in einer Formulierung vorgelegt, die für eine orale Verabreichung geeignet ist. Dabei kann der Wirkstoff selbst über ausreichende Polaritätseigenschaften verfügen, um beispielsweise in wässriger Lösung oral aufgenommen werden zu können. In einigen Ausführungsformen kann eine entsprechende Formulierung auch Zusatzstoffe wie Lösungsvermittler aufweisen, die eine orale Aufnahme des Wirkstoffs ermöglichen.

Eine als Rodentizid aktive Verbindung kann ein niedermolekularer Thrombininhibitor sein. Niedermolekulare Thrombininhibitoren hemmen Thrombin, auch in an Fibrin gebundener Form, typischerweise indem sie, beispielsweise als Substratanaloga, das katalytische Zentrum des Thrombins reversibel blockieren. In der Regel handelt es sich um substratanaloge Tripeptide, Derivate davon, oder von Benzamidin und Arginin abgeleitete Peptidomimetika. Niedermolekulare Thrombininhibitoren werden bzw. wurden therapeutisch zur Vorbeugung thromboembolischer Erkrankungen eingesetzt.

Ein Beispiel eines niedermolekularen Thrombininhibitors ist Melagatran, das mit hoher Affinität reversibel am aktiven Zentrum des Thrombins bindet. Eine Prodrug-Form, Ximelagatran (Exanta, Exarta, Exantan^{®}) kann am Menschen oral appliziert werden und wird schnell nach der Resorption in die wirksame Form Melagatran umgewandelt. Da eine Gabe über mehr als 35 Tage mit einem Risiko an Lebertoxizität verbunden ist, ist der Wirkstoff nicht zur Anwendung am Menschen zugelassen.

Dabigatran ist ein kompetitiver, reversibler und direkter Thrombinhemmer. Eine Prodrug-Form, Dabigatranetexilat (Pradaxa^{®}), das *in vivo* in Dabigatran umgewandelt wird, ist in der internationalen Patentanmeldung WO 03/ 074056 näher beschrieben. Dabigatran ist in der EU zugelassen zur Vorbeugung gegen die Bildung von Blutgerinnseln in den Venen nach elektivem chirurgischen Knie- oder Hüftgelenksersatz, sowie zur Schlaganfallvorbeugung bei Patienten mit Vorhofflimmern und Schlaganfallrisiko. Seine häufigste unerwünschte Arzneimittelwirkung (Nebenwirkung) am Menschen sind gastrointestinale Blutungen.

Ein weiteres Beispiel eines niedermolekularen Thrombininhibitors ist Argatroban (Argatra^{®}). Argatroban ist ein Argininderivat, das allerdings parenteral appliziert werden muss. Es kann jedoch in einer Mizellen-basierten Formulierung verabreicht werden, die auch oral aufgenommen wird. Eine solche Formulierung ist in der U. S.-Patentanmeldung US 5,679,690 beschrieben. Eine Lipidemulsion einer derartigen Verbindung ist auch in der Europäischen Patentanmeldung EP 0 608 828 offenbart. Ein festes Salz des Argatroban, das durch Präzipitation und Lyophilisation erhalten wird und für die orale Aufnahme geeignet sein soll, ist in der der U. S.-Patentanmeldung US 2009/ 0221637 offenbart. Auch Argatroban erhöht die Blutungsneigung, so dass Blutungen auch hier eine unerwünschte Arzneimittelwirkung sind.

In einigen Ausführungsformen ist eine der als Rodentizid im Schadnagetierköder aktive Verbindungen ein Thrombinrezeptor-Antagonist. Ein Thrombinrezeptor-Antagonist blockiert Protease-aktivierte Rezeptoren (PARs), die vorwiegend auf Blutplättchen zu finden sind und insbesondere durch Thrombin aktiviert werden. Dadurch wird die Wirkung des Thrombins auf die Blutplättchen und somit die Blutgerinnung gehemmt. In einigen Ausführungsformen ist der Thrombinrezeptor-Antagonist Atopaxar, auch als E5555 bekannt, gemäß EP 1 813 282. Atopaxar ist ein Hydrobromid mit dem IUPAC-Namen 1-(3-Tert-butyl-4-methoxy-5-morpholin-4-ylphenyl)-2-(5,6-diethoxy-4-fluoro-3-imino-1H-isoindol-2-yl)ethanon-hydrobromid. In zwei Phase II-Studien wurde eine Erhöhung der Leberenzyme beobachtet. In einer Studie beobachtete Blutungen wurden als statistisch nicht signifikant bezeichnet.

Ein Beispiel eines Thrombinrezeptor-Antagonisten gemäß US 2003/216437 ist Vorapaxar (Zontivity^{®}), auch als SCH 530348 bekannt. Dabei handelt es sich um N-[(3R,3aS,4S,4aR,7R,8aR, 9aR)-4-[(E)-2-[5-(3-Fluorophenyl)-2-pyridyl]vinyl]-3-methyl-1-oxo-3a,4,4a,5,6,7,8,8a,9,9a-decahydro-3H-benzo[f]isobenzofuran-7-yl]carbamat. Diese Verbindung ist in den USA für den Einsatz zur Verringerung des Risikos von Herzinfarkt, Schlaganfall und kardiovaskulärem Tod zugelassen. An Patienten, die zuvor einen Schlaganfall, eine transitorische ischämische Attacke (abgekürzt TIA) oder Blutungen im Kopf hatten, darf Vorapaxar auf Grund des hohen Blutungsrisikos im Kopf nicht eingesetzt werden. Allgemein sind eine unerwünschte Wirkung beim Einsatz von Vorapaxar am Menschen intrakranielle Blutungen.

In einigen Ausführungsformen ist eine der als Rodentizid im Schadnagetierköder aktiven Verbindungen ein Inhibitor von Faktor Xa. In einigen Ausführungsformen ist der Inhibitor von Faktor Xa Rivaroxaban (Xarelto^{®}; gemäß WO 01/47919). Rivaroxaban ist ein oral wirksames Antikoagulans. Wie für andere hier genannte Verbindungen sind bei Anwendung am Menschen unerwünschte Arzneimittelwirkungen von Rivaroxaban akute, klinisch relevante Blutungen.

Ein Beispiel eines weiteren direkten Inhibitors des Faktor Xa gemäß US 2003/191115 und WO 03/026652 ist Apixaban (Eliquis^{®}), das ebenfalls oral wirksam ist. Es ist in der EU zur Prophylaxe von venösen Thromboembolien nach elektiven orthopädischen Operationen sowie zur Prävention von ischämischen Schlaganfällen und systemischen Embolien bei Erwachsenen mit nicht-valvulärem Vorhofflimmern zugelassen. Daneben ist es in der EU zur Therapie und Rezidivprophylaxe von Lungenembolien und tiefen Venenthrombosen zugelassen. Unerwünschte Arzneimittelwirkung am Menschen ist auch hier das Risiko schwerer Blutungen.

Ein Beispiel für einen Faktor Xa-Inhibitor gemäß EP 2 343 290, der oral applizierbar ist, ist Edoxaban (Lixiana^{®}, Savaysa^{®}). In der Europäischen Patentanmeldung EP 2 374 456 ist Edoxaban näher erläutert. Blutungen wie Nasenbluten oder schwere nicht-menstruale vaginale Blutungen sind bekannte unerwünschte Arzneimittelwirkungen.

Ein Beispiel für einen Faktor Xa-Inhibitor gemäß WO 01/64642 und WO 01/64643, der oral applizierbar ist, ist Betrixaban (N-(5-Chlorpyridin-2-yl)-2-([4-(N,N-dimethylcarbamimidoyl)-benzoyl]amino)-5-methoxybenzamid). Auch für Betrixaban als Medikament am Menschen wurden Blutungen als unerwünschte Wirkung beobachtet.

Darexaban (N-(3-Hydroxy-2-{[4-(4-methyl-1,4-diazepan-1-yl)-benzoyl]amino}phenyl)-4-methoxybenzamid) ist ein Beispiel eines Inhibitors des Faktor Xa gemäß EP 1 336 605, dessen Entwicklung als Arzneimittel aufgrund von Blutungen als unerwünschte Arzneimittelwirkung nicht weiterverfolgt wurde.

Otamixaban (Methyl (2R,3R)-2-{3-[amino(imino)methyl]benzyl}-3-{[4-(1-oxidopyridin-4-yl)benzoyl]amino}butanoat) ist ein Beispiel für einen Inhibitor des Faktors Xa gemäß WO 97/24118, dessen Entwicklung als Arzneimittel nicht weiterverfolgt wurde.

Ein im Rahmen der vorliegenden Offenbarung geeigneter Wirkstoff kann auch ein Antagonist des Adenosindiphosphat-Rezeptors P2Y12 auf den Blutplättchen sein. Durch Bindung eines solchen Antagonisten wird die Bindung von ADP an diesen Rezeptor blockiert, was eine Hemmung der Plättchenaggregation zur Folge hat.

Ein Beispiel eines Antagonisten des P2Y12-ADP-Rezeptors ist Ticlopidin (Tiklyd^{®}; gemäß US 4,051,141 und US 4,591,592). Ticlopidin ist ein Prodrug, das *in vivo* in einen aktiven Metaboliten umgewandelt wird.

Clopidogrel (Iscovera, Plavix^{®}) ist ein oral verabreichbarer Thrombozytenaggregationshemmer gemäß EP 0 099 802 und US 4,529,596. Das U.S.-Patent US 4,847,265 beschreibt Clopidogrel. Auch Clopidogrel ist ein Prodrug, das erst *in vivo* in einen aktiven Metaboliten umgewandelt wird, der als nicht-kompetitiver irreversible Antagonist des P2Y12-ADP-Rezeptors wirkt. Im Menschen wird das Maximum der Aggregationshemmung nach oraler Gabe von Ticlopidin oder Clopidogrel nach 4 bis 6 Tagen erreicht. Zu unerwünschten Arzneimittelwirkungen zählen Blutungen wie Nasenbluten, Magen- oder Darmblutungen, Hämatome oder Blut im Urin.

Ein weiteres Beispiel für einen Antagonisten des P2Y12-ADP-Rezeptors ist eine Verbindung gemäß dem Europäischen Patent EP 0 099 802 oder dem U. S.-Patent US 5,288,726, nämlich Prasugrel ((RS)-[5-[2-Cyclopropyl-1-(2-fluorphenyl)-2-oxoethyl]-6,7-dihydro-4H-thieno[3,2-c]pyridin-2-yl]acetat). Das Europäische Patent EP 1 298 132 und das U.S.-Patent US 6,693,115 beschreiben Prasugrel. Ebenso wie Ticlopidin und Clopidogrel ist Prasugrel ein Prodrug, das *in vivo* in einen Thiol enthaltenden aktiven Metaboliten umgewandelt wird. Auch Prasugrel ist ein nicht-kompetitiver irreversibler Antagonist des P2Y12-ADP-Rezeptors auf Blutplättchen. Blutungen als unerwünschte Arzneimittelwirkung beim Menschen sind auch hier bekannt. Als Risikogruppen für Blutungen bei Behandlung mit Prasugrel und Clopidogrel wurden unter anderem > 75 jährige Patienten und Patienten mit einem Körpergewicht < 60 kg identifiziert.

Ein weiteres Beispiel für einen Antagonisten des P2Y12-ADP-Rezeptors ist eine Verbindung gemäß der Internationalen Patentanmeldung WO 2000/34283, nämlich Ticagrelor (Brilinta^{®}, Brilique^{®}, Possia^{®}). Dieser Antagonist ist ein reversibler Antagonist des P2Y12-ADP-Rezeptors. Schwerwiegende und leichte Blutungen sind auch für Ticagrelor als unerwünschte Wirkungen beim Einsatz am Menschen bekannt.

Ein weiteres Beispiel für einen typischerweise oral verabreichbaren Thrombozytenaggregationshemmer ist das Derivat des Schlangengifts der Sandrasselotter, Tirofiban (Aggrastat^{®}), ein Antagonist des GPIIb/IIIa-Rezeptors auf Blutplättchen. Eine solche Verbindung ist ebenfalls als Rodentizid gemäß der vorliegenden Offenbarung geeignet. Die internationale Patentanmeldung WO 99/38827beschreibt diese Verbindung. Diese muss allerdings parenteral verabreicht werden.

In einigen Ausführungsformen ist ein geeigneter Antagonist des GPIIb/IIIa-Rezeptors eine Verbindung gemäß der Internationalen Patentanmeldung WO 90/15620, nämlich Eristicophin, Cotiarin, Crotatroxin, Cerastin, Durissin, Horridin, Ruberin, Lachesin, Basilicin, Lutosin, Molossin, Oreganin, Viridin, Tergeminin oder Barbourin.

Ein Beispiel eines entsprechenden GPIIb/IIIa-Rezeptorantagonisten ist Arginin-Glycin-Aspartat-Mimetikum wie beispielsweise das Peptid Eptifibatid (Integrilin^{®}). Dieser Wirkstoff muss in der kommerziell erhältlichen Form allerdings parenteral verabreicht werden.

Ein Beispiel eines GPIIb/ IIIa-Rezeptorantagonisten nach US 5,721,366 ist Orbofiban, Ethyl N-{[(3S)-1-(4-carbamimidoylphenyl)-2-oxo-3-pyrrolidinyl]carbamoyl}-β-alaninat.

Ein Beispiel eines GPIIb/ IIIa-Rezeptorantagonisten nach WO 95/14683 ist Roxifiban (DMP 754, MK 0853, XJ 754, Lumaxis^{®}), Methyl-N3-[2-{3-(4-formamidinophenyl)-isoxazolin-5-(R)-yl}-acetyl]-N2-(n-butyloxycarbonyl)-2,3-(S)- diaminopropionat.

Ein Beispiel eines GPIIb/IIIa-Rezeptorantagonisten nach EP 0 656 348 ist Sibrafiban (Ro 48-3657, Xubix^{®}). Zu den häufigsten unerwünschten Arzneimittelwirkungen von Sibrafiban am Menschen zählen Blutungen.

Ein Beispiel eines GPIIb/IIIa-Rezeptorantagonisten nach WO 93/19046 ist Tirofiban ((S)-2-(Butylsulfonamino)-3-(4-[4-(piperidin-4-yl)butoxy]phenyl)-propansäure, Aggrastat^{®}). Als häufige unerwünschte Arzneimittelwirkung am Menschen sind leichte Blutungen bekannt, es können auch schwere Blutungen auftreten.

Die einzigartige Wirksamkeit von bisher als Rodentizid eingesetzten Cumarinen und Indandion-Verbindungen beruht auf deren oraler Aufnahme mit einem verzögerten Einsetzen der Intoxikationswirkung. Anders gesagt wird keine akute Toxizität beobachtet. Damit ist für das Nagetier kein zeitlicher Bezug zwischen Aufnahme der Verbindung und Einsetzen der Wirkung herstellbar. Auch die hier offenbarten Schadnagetierköder beruhen in der Regel darauf, dass die enthaltenen Wirkstoffe in einer Dosierung bereitgestellt werden, bei der keine akute Toxizität auftritt. Die hier offenbarten Schadnagetierköder sind zur Bekämpfung von Wildnagern grundsätzlich geeignet, sobald der eingesetzte Wirkstoff einem Nagetier oral zuführbar ist. Ohne sich auf eine bestimmte Theorie einzuschränken, sind die hier offenbarten Schadnagetierköder zur Bekämpfung von Wildnagern aus dem Grund besonders geeignet, dass eine eingesetzte Verbindung eine lange Halbwertszeit wie beispielsweise mehrere Tage hat und eine progressive Akkumulation in den jeweiligen Bereich der LD 100 erfolgen kann. Auch ist die jeweilige LD 100 in der Regel durch normale Futtermengen des Nagers wie einer Ratte zu erreichen. Des Weiteren ist kein Ceiling-Effekt bekannt, d.h. die intestinalen Resorptionsmechanismen sind als im relevanten Rahmen nicht erschöpflich anzusehen. Schließlich sind die für die hier offenbarte Verwendung genannten Wirkstoffe mit einem Köder mischbar, der schmackhaft sein kann, oder es ist ein Aufbringen auf einen Köder ohne Geschmacksbeeinträchtigung möglich. Ob es zusätzlich zu chronischen Toxizitäten kommt oder kommen kann, ist für die hier beschriebene Wirkung als Rodentizid in der Regel ohne Belang.

Angemerkt sei des Weiteren ohne sich auf eine bestimmte Theorie einzuschränken, dass eine einmalige versehentliche Ingestion, beispielsweise eines Köders, beim Menschen nicht zum Tode führt. Neben der großen Differenz im Körpergewicht und damit der absoluten letalen Dosis zwischen z.B. Ratte und Mensch ist in der Regel ein Köder auch nicht für eine absichtliche Beibringung mit Todesfolge geeignet, da sich eine letale Wirkung am Menschen mit Dimensionen eines Nagetierköders typischerweise nicht praktisch verwirklichen lässt. Der entsprechende Wirkstoff müsste in großer Menge auf für den Menschen ungeeignetem Träger wiederholt eingenommen werden, um eine letale Dosis zu erreichen.

In einigen Ausführungsformen kann ein hier offenbarter Schadnagetierköder mit einer Zusammensetzung kombiniert werden, die ein Getreidemehl, eine Getreidekleie, ein Geliermittel, einen Zucker, ein Öl, einen Emulgator und ein Feuchthaltemittel enthält, wie beispielsweise in der internationalen Patentanmeldung WO 2014/186885 beschrieben. In einigen Ausführungsformen kann ein hier offenbarter Wirkstoff mit einer Zusammensetzung kombiniert werden, die wie beispielsweise in WO 2014/186885 beschrieben ein Getreidemehl, eine Getreidekleie, ein Geliermittel, einen Zucker, ein Öl, einen Emulgator und ein Feuchthaltemittel enthält.

In einigen Ausführungsformen kann ein hier offenbarter Schadnagetierköder in verpackter Form vorliegen. Eine solche Verpackung kann beispielsweise ein Gehäuse, eine Abdeckung, sowie eine dazwischen liegende Höhlung aufweisen wie in der internationalen Patentanmeldung WO 2003/061376 beschrieben. In einigen Ausführungsformen wird ein hier offenbarter Wildnagetierköder in Verbindung mit einer Köderstation eingesetzt, beispielsweise wie sie kommerziell erhältlich ist. Ein entsprechender Köder kann beliebige als Nagetiernahrung geeignete Stoffe enthalten, solange entsprechende Stoffe nicht selbst den Tod des entsprechenden Nagetiers innerhalb von wenigen Tagen zu Folge haben. Ein illustratives Beispiel ist ein Köder auf Getreidebasis.

Ein hier offenbarter Wildnagetierköder kann beispielsweise Samen und / oder Getreide enthalten. Der Wildnagetierköder kann beispielsweise in Form von Granulat (engl. Pellets), in Form von verpacktem Getreide oder verpacktem Granulat oder als Köderblock vorgelegt werden. In einigen Ausführungsformen kann eine hier offenbarte Wirkstoffkombination in einem Köderblock enthalten sein, der ein polymeres Bindemittel in Form eines Polymers auf Basis eines Acrylsäureesters und Acrylnitril enthält, wie er beispielsweise in der internationalen Patentanmeldung WO 2014/064272 beschrieben ist.

Das Blutgerinnungssystem der Ratte und des Menschen ähneln sich sehr. Auch beim Menschen können beispielsweise Cumarinüberdosierungen zu lebensbedrohlichen Blutungen führen. Eine sehr sorgfältige Antikoagulantienführung ist daher Voraussetzung für ihren sicheren therapeutischen Einsatz. Es hat sich des Weiteren herausgestellt, dass eine ursprünglich erhoffte Dissoziation von Hämostase-blockierender Wirkung der konventionellen neuen direkten Antikoagulantien (z.B. Dabigatran) und Blutungskomplikationen beim Menschen leider nicht so wie erwartet eintritt. Es besteht mit diesen neuen oralen Antikoagulantien weiterhin eine Dosis-abhängig zunehmende Rate schwerer und tödlicher Blutungen. Ohne die Absicht, sich an eine Theorie zu binden, erklärt sich daher, dass die hier offenbarten Schadnagetierköder in der Bekämpfung von Schadnagetieren wirksam sind. Darüber hinaus sind für die als in den erfindungsgemäßen Schadnagetierködern enthaltenen offenbarten Verbindungen keine alternativen Aktivierungswege im Nagerorganismus bekannt, so dass mit einer Resistenzentwicklung nicht zu rechnen ist.

Die Nennung oder Diskussion eines zuvor veröffentlichten Dokuments in dieser Beschreibung sollte nicht notwendigerweise als Anerkenntnis verstanden werden, dass ein solches Dokument zum Stand der Technik zählt oder Allgemeinwissen des Fachmanns darstellt.

Die Ausführungsformen des Schadnagetierköders sind im nachfolgenden Patentanspruch wiedergegeben.

## Patentansprüche

1. Schadnagetierköder, enthaltend zwei oder mehr Verbindungen ausgewählt aus der Gruppe bestehend aus den Klassen Thrombininhibitoren, Thrombinrezeptor-Antagonisten, Faktor Xa-Inhibitoren, Antagonisten des ADP-Rezeptors P2Y12 und Antagonisten des GPIIb/IIIa-Rezeptors,
wobei der Thrombininhibitor wenigstens eine Verbindung ausgewählt aus der Gruppe bestehend aus Melagatran, Ximelagatran, Dabigatran, Dabigatranetexilat und Argatroban ist,
wobei der Thrombinrezeptor-Antagonist wenigstens eine Verbindung ausgewählt aus der Gruppe bestehend aus Atopaxar und Vorapaxar ist,
wobei der Inhibitor von Faktor Xa wenigstens eine Verbindung ausgewählt aus der Gruppe bestehend aus Rivaroxaban, Apixaban, Edoxaban, Betrixaban, Darexaban und Otamixaban ist,
wobei der Antagonist des ADP-Rezeptors P2Y12 wenigstens eine Verbindung ausgewählt aus der Gruppe bestehend aus Ticlopidin, Clopidogrel, Prasugrel und Ticagrelor ist,
wobei der Antagonist des GPIIb/IIIa-Rezeptors wenigstens eine Verbindung ausgewählt aus der Gruppe bestehend aus Eristicophin, Cotiarin, Crotatroxin, Cerastin, Durissin, Horridin, Ruberin, Lachesin, Basilicin, Lutosin, Molossin, Oreganin, Viridin, Tergeminin, Barbourin, Eptifibatid, Orbofiban, Roxifiban, Sibrafiban und Tirofiban ist.

## Claims

1. Rodent bait containing two or more compounds selected from the group consisting of the classes thrombin inhibitors, thrombin receptor antagonists, factor Xa inhibitors, antagonists of the ADP receptor P2Y12, and antagonists of the GPIIb/IIIa receptor,
wherein the thrombin inhibitor is at least one compound selected from the group consisting of melagatran, ximelagatran, dabigatran, dabigatran etexilate, and argatroban,
wherein the thrombin receptor antagonist is at least one compound selected from the group consisting of atopaxar, and vorapaxar,
wherein the inhibitor of Factor Xa is at least one compound selected from the group consisting of rivaroxaban, apixaban, edoxaban, betrixaban, darexaban, and otamixaban,
wherein the antagonist of the ADP receptor P2Y12 is at least one compound selected from the group consisting of ticlopidine, clopidogrel, prasugrel, and ticagrelor,
wherein the antagonist of the GPIIb/IIIa receptor is at least one compound selected from the group consisting of eristicophin, cotiarin, crotatroxin, cerastin, durissin, horridin, ruberin, lachesin, basilicin, lutosin, molossin, oreganin, viridin, tergeminin, barbourin, eptifibatid, orbofiban, roxifiban, sibrafiban, and tirofiban.

## Revendications

1. Appât pour rongeurs nuisibles contenant deux composés ou plus sélectionnés dans le groupe constitué des catégories suivantes : inhibiteurs de thrombine, antagonistes de récepteurs de thrombine, inhibiteurs du facteur Xa, antagonistes du récepteur ADP P2Y12 et antagonistes du récepteur GPIIb/IIIa,
dans lequel l'inhibiteur de thrombine est au moins un composé sélectionné dans le groupe constitué du melagatran, du ximelagatran, du dabigatran, du dabigatranetexilat et de l'agratroban,
dans lequel l'antagoniste du récepteur de thrombine est au moins un composé sélectionné dans le groupe constitué de l'atopaxar et du vorapaxar,
dans lequel l'inhibiteur du facteur Xa est au moins un composé sélectionné dans le groupe constitué du rivaroxaban, de l'apixaban, de l'édoxaban, du betrixaban, du darexaban et de l'otamixaban,
dans lequel l'antagoniste du récepteur ADP P2Y12 est au moins un composé sélectionné dans le groupe constitué de la ticlopidine, le clopidogrel, le prasugrel et le ticagrelor,
dans lequel l'antagoniste du récepteur GPIIb/IIIa est au moins un composé sélectionné dans le groupe constitué de : eristicophine, cotiarine, crotatroxine cerastine, durissine, horridine, rubérine, lachesine, basilicine, lutosine, molossine, oreganine, viridine, tergeminine, barbourine, eptifibatide, orbafiban, roxifiban, sibrafiban et tirofiban.
